# EUROPEAN PATENT APPLICATION

(11) **EP 2 191 867 A1**
(43) Date of publication of application: **02.06.2010**
(21) Application number: 08020592.5
(22) Date of filing: 27.11.2008
(51) Int. Cl.: A61Q 5/08, A61K 8/22, A61K 8/64, A61K 8/98

(54) **Bleaching/Highlighting composition**

(71) Applicant: KPSS-Kao Professional Salon Services GmbH, 64297 Darmstadt (DE)
(72) Inventor: Pratt, Dominic, 64572 Büttelborn (DE); Schäfer, Sabine, 65428 Rüsselsheim (DE)
(74) Representative: Grit, Mustafa

(57) **Abstract**

The present invention relates to a bleaching composition for keratin fibres, especiafly human hair, comprising at least one compound with bleaching and/or highlighting effect wherein a water soluble protein selected from water soluble silk protein, Water soluble silk protein hydrolysate and their derivatives is added. The object of the present invention is an aqueous composition for bleaching hair comprising three parts which are mixed prior to application onto hair and which are
a- a substantially anhydrous composition comprising at least one compound with bleaching effect,
b- an aqueous composition comprising at least one oxidizing agent, and
c- a composition comprising at least one compound selected from water soluble silk protein, water soluble silk protein hydrolysate and of their derivatives.

## Description

The present invention relates to bleaching composition for keratin fibres, especially human hair, comprising at least one compound with bleaching and/or highlighting effect wherein a water soluble protein selected from water soluble silk protein, water soluble silk protein hydrolysate and their derivatives is added.

Hair bleaching is a common practice for ages. It is based on oxidative decomposition of hair colour, which is usually done using peroxide or peroxide releasing compounds such as persulfates. Due to highly irritating potential of these bleaching ingredients and dustiness of powder compositions, it is preferred to provide granular composition where dust is reduced by agglomerating small particles into granulates using various binding agents. Most popular binding agent is mineral oil, which was the subject matter of EP 560 088 B1. Furthermore, EP 778 020 A1 suggests the use of oil and wax compounds or their mixtures for preparation of suspensions.

The bleaching of human hair customarily consists of a process with the following steps: Homogenous mixing of a water-free preparation, preferably a powder, comprising at least one compound with a bleaching and/or highlighting effect, in particular a solid peroxide salt, preferably ammonium, potassium and/or sodium persulfate or earth alkali peroxide, with an aqueous hydrogen peroxide composition, application of this composition onto the hair, and rinsing after bleaching is completed. It is known that bleaching components are chemically active on hair and damages hair which is afterwards difficult to repair. Damaged hair brings further difficulties especially in daily care as it does not behave any more as natural hair does and people having bleached hair are obliged to use special conditioning and/or daily care products in order to keep their hair looking attractive. This is certainly time consuming and especially expensive and, therefore, there is a great need of new bleaching compositions with reduced degree of hair damage. It should be noted that damaging effects of bleaching compositions are unavoidable and, therefore, the objective of present invention is reducing the damaging effect of commonly used bleaching agents.

The damage level of hair is often measured by its cysteic acid since cysteic acid is formed due to hair fibre degradation. The higher the hair damage, the higher cysteic acid level in hair.

It has been surprisingly found out by the inventors of the present invention that when a bleaching composition is added at least one water soluble silk protein and/or at least one water soluble silk protein hydrolysate and/or at least one of their derivatives, damaging effect of bleaching composition on hair is noticeably reduced.

Accordingly, the first object of the present invention is aqueous composition for bleaching hair comprising three parts which are mixed prior to application onto hair and which are
a- a substantially anhydrous composition comprising at least one compound with bleaching effect,
b- an aqueous composition comprising at least one oxidizing agent, and
c- a composition comprising at least one compound selected from water soluble silk protein, water soluble silk protein hydrolysate and of their derivatives.

Further object of the present invention is a process for bleaching hair wherein an aqueous bleaching composition is prepared by mixing three compositions wherein
a- a substantially anhydrous composition comprising at least one compound with bleaching effect,
b- an aqueous composition comprising at least one oxidizing agent, and
c- a composition comprising at least one compound selected from water soluble silk protein, water soluble silk protein hydrolysate and of their derivatives,
is applied onto hair and after processing of 5 to 45 min at a temperature in the range of 20 to 45°C is rinsed off from hair.

Another object of the present invention is use of water soluble silk protein, water soluble silk protein hydrolysate and of their derivatives in a bleaching composition for reducing hair damage.

Still further object of the present invention is a kit for bleaching hair which comprises 3 compositions wherein
a- a substantially anhydrous composition comprising at least one compound with bleaching effect,
b- an aqueous composition comprising at least one oxidizing agent, and
c- a composition comprising at least one compound selected from water soluble silk protein, water soluble silk protein hydrolysate and of their derivatives.

According to the present invention, the first composition, a substantially anhydrous composition, comprises at least one compound with bleaching and/or highlighting effect. Suitable compounds are in general peroxides. Useful as such are in particular persulfates such as sodium and potassium persulfate, ammonium persulfate, earth alkali peroxides such as magnesium peroxide, melamine peroxide or urea peroxide or phtholimidoperoxyhexanoic acid, and mixtures thereof. The proportion of peroxides is at least 5%, preferably in the range of 20 to 80%, more preferably 25 to 60% and most preferably 30 to 55% by weight, calculated to total of each composition.

According to the invention, the substantially anhydrous composition can also comprise 0.1 % to 10% by weight, calculated to total of each composition, at least one ammonium salt. Suitable ammonium salts are ammonium carbonate, ammonium hydrogen carbonate, ammonium carbamate, ammonium chloride, ammonium sulfate, ammonium phosphates, ammonium nitrate, ammonium bromide, ammonium iodide, ammonium thiosulfate, ammonium molybdate, ammonium vanadate, ammonium sulfamate, ammonium citrate, ammonium salicylate, ammonium valerate, ammonium tartarate, ammonium benzoate, ammonium acetate, ammonium formiate and ammonium lactate. Compositions may also comprise mixture or ammonium salts.

Preferred thereof are the ammonium phosphates, such as ammonium dihydrogen phosphate, ammonium hydrogen phosphate, diammonium sodium phosphate, sodium ammonium hydrogen phospahe, ammonium disodium phosphate, as well as ammonium chloride, ammonium sulphate, diammonium hydrogen citrate, ammonium carbonate, ammonium hydrogen carbonate preferably in an amount from 0.1 % to 10% by weight, calculated to total composition prior to mixing with oxidizing lotion.

As known from EP 609 796 A2, the ammonium compounds can also be used as sole bleaching agent in respectively higher amounts.

The total proportion of the compounds with bleaching and/or highlighting effect preferably ranges from 5% to 85%, preferably 20% to 80%, more preferably 25 to 70% and most preferably 30 to 60% by weight calculated to total of each composition.

In principal, substantially anhydrous composition comprising at least one compound with bleaching effect can be in any from such as solution, suspension, powder, etc. Preferred forms are suspension and powder and especially preferred from is powder form and in particular a dust free powder whose preparation is well known in the literature.

In addition to the active component, substantially anhydrous bleaching compositions also comprise the components customarily used in such compositions: In particular inert pulverulent carrier materials, these are for example, pyrogenic silicium dioxide, starch powder, etc., alkalizing agents, such as sodium metasilicate, surface-active substances, binding agents, etc. In order to avoid repetition, reference is made to the respective standard literature, for example, K. Schrader and A. Domsch, "Cosmetology - Theory and Practice (2005, Verlag für Chemische Industrie), pages 142 to 151.

In a preferred embodiment of the present invention, substantially anhydrous bleaching composition of the present invention is in powder form and in particular in dust free powder form and comprises oily lipophilic ingredients such as vegetable oils, for example, jojoba oil or any other; petrolatum liquid paraffins, especially paraffinum perliquidum and paraffinum subliquidum; silicone oils; hydrophobic fatty acid esters such as octyl palmitate, isocetyl palmitate, isopropyl palmitate and octyl stearate, C₁₀- to C₃₆-fatty acid triglycerides, as well as their mixtures. In the case that the use is wished among those the most preferred ones are silicone oils, jojoba oil, fatty acid esters, paraffin oils, combinations of fatty acid esters and paraffin oils. Fatty acid esters and/or paraffin oils and/or silicone oils are particularly preferred. Concentration of these oily lipophilic compounds are used in a total amount of about 0.1 to 50% by weight, preferably from 1 to 40% by weight, and more preferably from 2 to 35% by weight, calculated to total of anhydrous composition.

In principal any silicone oil is useful as a lipophilic compound. Preferred are dimthicones, dimethiconols and arylated silicones as a lipophilic ingredient at a concentration range of 0.1 to 50%, preferably 0.5 to 40% more preferably 1 to 35% and most preferably 2.5 to 30% by weight calculated to total composition prior to mixing with oxidizing lotion. Non-limiting suitable examples are dimethicones with various viscosity available from Dow Corning under the trade name DC 200, arylated silicones such as phenyl methicone, phenyl trimethicone, diphenyl dimethicone, diphenylsiloxy phenyl trimethicone, tetramethyl tetraphenyl trisiloxane, triphenyl trimethicone, tetramethly tetraphenyl trisiloxane and pentaphenyl trimethyl trisiloxane.

Further, in another preferred form of the invention substantially anhydrous composition for bleaching hair comprises polymers from the group consisting of cellulose polymer compounds, alginate, polysaccarides and acrylic acid polymers, preferably methyl cellulose compounds, ethyl cellulose compounds, hydroxyethylcellulose compounds, methylhydroxyethylcellulose compounds, methylhydroxypropylcellulose compounds, carboxymethyl cellulose compounds, alginic acids, sodium alginates, ammonium alginates, calcium alginates, gum arabic, guar gum or xanthan gum, or acrylic acid polymers with molecular weights from about 1,250,000 to 4,000,000, alone or in combination with each other. The polymers are used in a total amount of 0.1 to 15 %, preferably from 0.2 to 10 %, and more preferably in an amount of from 0.5 to 7.5% by weight, calculated to total of each composition.

Substantially anhydrous bleaching and/or highlighting composition can also comprise cationic polymers as conditioning and/or thickening agents. Those are cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, it has been found suitable those cationic polymers known with their CTFA category name Polyquaternium. Typical examples of those Polyquaternium 1, Polyquaternium 2, Polyquaternium 4, Polyquaternium 5, Polyquaternium 6, Polyquaternium 7, Polyquaternium 8, Polyquaternium 9, Polyquaternium 10, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

As well those polymers known with their CTFA category name Quaternium are suitable. Those are for example Quaternium-8, Quaternium-14, Quaternium-15, Quaternium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-82, Quaternium-83 and Quaternium-84.

Typical concentration range for any of the cationic polymers mentioned above can be 0.01 - 7.5% by weight, preferably 0.05 - 5% by weight and more preferably 0.1 - 2.5% by weight, calculated to total composition prior to mixing with an oxidizing agent.

It has been observed in practice that heat may develop when substantially anhydrous composition is mixed with one or more aqueous compositions and especially aqueous oxidizing composition. In order to overcome such heat development, substantially anhydrous composition of the present invention preferably comprises at least one calcium salt. Suitable salts are practically any calcium salt but preferably selected from calcium aluminium borosilicate, calcium aspartate, calcium benzoate, calcium acetate, calcium carbonate, calcium citrate, calcium dihydrogen phosphate, calcium fluoride, calcium hydroxide, calcium lactate, calcium monofluorophosphate, calcium oxide, calcium phosphate, calcium propionate, calcium pyrophosphate, calcium sulphate, calcium nitrate, calcium salicylate, calcium silicate, hydrate, calcium tartarate, tricalcium phosphate, calcium chloride, calcium iodide and calcium bromide

The most preferred are calcium carbonate, calcium dihydrogen phosphate, calcium fluoride, calcium hydroxide, calcium oxide, calcium phosphate, calcium pyrophosphate, calcium sulphate, calcium nitrate, calcium chloride, calcium iodide and calcium bromide. Calcium sulphate is particularly preferred because of its outstanding effect.

Concentration of at least one calcium salt in substantially anhydrous composition of the present invention is between 0.1 and 20%, preferably between 0.5 and 15%, more preferably between 0.75 and 10% and most preferably between 1 and 7.5% by weight calculated to total composition prior to mixing with oxidizing lotion.

Substantially anhydrous bleaching composition of the present invention may comprise at least one dialkyl carbonate of general formula

R₁ O C(O) O R₂

where R₁ and R₂ are independent from each other linear or branched saturated alkyl chains with 6 to 22 C atoms.

Preferred at least one dialkyl carbonate is selected from di(caprylyl) carbonate and di(ethylhexyl) carbonate.

Concentration of at least dialkyl carbonate may vary between 0.1 and 30% by weight calculated to total composition prior to mixing with an oxidizing agent.

Substantially anhydrous bleaching compositions of the present invention can comprise synthetic mica coated with metal oxide or oxides having a volume particle size distribution in the range of 1 to 750 µm. Use of synthetic mica coated with metal oxide or oxides mainly in decorative cosmetics is disclosed in an international patent application of Sun Chemical Corporation published with a number WO 2005/065632 A1. In the document synthetic mica and coated synthetic mica with at least one metal oxide or oxides is disclosed in detail. The content of the document is included herewith by reference.

Suitable metal oxide or oxides for coating synthetic mica are titanium dioxide, chromium oxide, ferric oxide or mixtures thereof. In the present invention the preferred is synthetic mica coated with titanium dioxide. Such materials are commercially available from Sun Chemical Corporation and Merck (Timiron Synwhite 40) and known with their INCI names Synthetic Fluorphologopite

The volume particle size distribution of synthetic mica coated with a metal oxide or oxides is in the range of 1 to 750 µm, preferably 1 to 250 µm, more preferably 1 to 100 µm and most preferably 5 to 95 µm. The particle sizes referred are relating to the volume particle size distribution meaning that particles found in the coated synthetic mica having volume particle size in the given ranges.

Concentration of synthetic mica coated with at least metal oxide or oxides is from 0.01 to 20%, preferably 0.1 to 15%, more preferably 0.25 to 10% and most preferably 0.5 to 55% by weight calculated to total composition prior to mixing with an oxidizing agent.

Interestingly, it has been observed that the mixture of substantially anhydrous powder composition comprising at least one bleaching and/or highlighting agent and synthetic mica coated with at least one metal oxide or oxides with a particle size as mentioned above and a composition comprising at least one oxidizing agent look very homogeneous, shiny and easy to apply onto hair compared to a mixture which does not comprise synthetic mica.

The average particle size of the dust free bleaching powder composition according to the invention is generally below 1 mm, preferably below 500 µm, more preferably less than 400 µm and in particular about 25 to about 100 µm, thus ensuring excellent processing capability, i.e. miscibility with an aqueous hydrogen peroxide solution prior to application onto human hair.

The powder composition can be produced with processes such as by mixing the powdery ingredients first and subsequently adding lipophilic ingredient(s) and by the fluidized bed method. In the fluidized bed method, powder ingredients are mixed in a vessel and made flowing by inletting an air flow which may be heated (preferred when using waxy component) or carried out at room (ambient) temperature and while the powder mix freely "flowing" lipophilic ingredient and/or mixture with any other liquid component is sprayed from a nozzle mounted above the powder batch.

The second composition is an aqueous composition and comprises at least one oxidizing agent. The oxidizing agents suitable are hydrogen peroxide, urea peroxide, melamin peroxide or perborate salts. The most preferred is hydrogen peroxide. Such composition comprises 2 to 12% by weight at least one oxidizing agent preferably hydrogen peroxide and is either a solution or in the form of an emulsion. It may further comprise those substances customarily found in oxidizing compositions such as chelating agents such as EDTA and/or its salts, compounds which stabilize especially hydrogen peroxide in aqueous compositions, fragrance, acidifying agents to adjust the pH, especially phosphoric acid and/or its salts. pH of the second aqueous composition is in the range of 2 to 7, preferably 2.5 to 6 and more preferably 2.5 to 5.

The third composition comprises at least one compound selected from water soluble silk protein, water soluble silk protein hydrolysate and of their derivatives. Suitable ones are silk obtained from various resources, silk powder so far proteins are soluble in water, hydrolysed silk protein, cocoyl hyrolysed silk, Isostearoyl hydrolysed silk, cocodimonium hydroxypropyl hydrolysed silk, hydroxypropyltrimonium hydrolysed silk, isostearoyl hydrolysed silk, laurdimonium hydroxypropyl hydrolysed silk, MEA hydrolysed silk, PG-hydrolysed silk, sodium cocoyl hydrolysed silk, sodium lauroyl hadrolysed silk, sodium stearoyl hydrolysed silk, steartrimonium hydroxypropyl hydrolysed silk. All of the above mentioned suitable examples within the meaning of the present invention are commercially available from various suppliers. Especially preferred is hydrolysed silk protein commercially available under the trade name Cosi Silk Soluble from Cosnaderm GmbH.

Concentration of at least one compound selected from water soluble silk protein, water soluble silk protein hydrolysate and of their derivatives is in the range of 0.001 to 2.5%, preferably 0.005 to 2% more preferably 0.01 to 1.5 and most preferably 0.05 to 1% by weight, calculated to total of the third aqueous composition.

The third composition is preferably an aqueous composition and can be in the form of a solution, thickened composition, gel, emulsion or a suspension. It may even be a product dispensed from a pressurised container including any one of the above mentioned type of preparations together with at least one propellant is a suitable packaging.

The third composition preferably comprises additionally at least one hair conditioning compound. Hair conditioning compound is preferably selected from non-ionic substances, oil or oily substances, cationic compounds.

Non-ionic conditioning agents can be polyethyleneglycol mono or di fatty acid esters having general formula

R₁ CO (O CH₂ CH₂)ₙ OH
or
R₁ CO (O CH₂ CH₂)ₙ O OC R₂

wherein R₁ and R₂ are independent from each other saturated, unsaturated or branched or non-branched alkyl chain with 7 to 21 C atoms and n is typically 2 - 100.

Suitable cationic conditioning compounds are those carrying a quaternary ammonium group or a cationizable group and selected from cationic polymers, cationic surfactants and quaternary ammonium or amino silicones. Suitable cationic polymers are those of best known with their CTFA category name Polyquaternium as disclosed above for water free bleaching and/or highlighting composition as well as those polymers known with their CTFA category name Quaternium and disclosed above for water free bleaching and/or highlighting composition.

The third composition and also water free bleaching composition of the present invention can comprise an organopolysiloxane wherein at least one silicium atom is linked to an alkylene group having a hetero-atom, in particular a nitrogen atom, with a poly-(N-acyl alkyleneimine) units of the formula 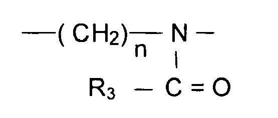 wherein n is a number from 1 to 5 and R₃ is hydrogen, a C₁-C₁₂-alkyl or cycloalkyl, aralkyl or aryl group as a conditioning agent. Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 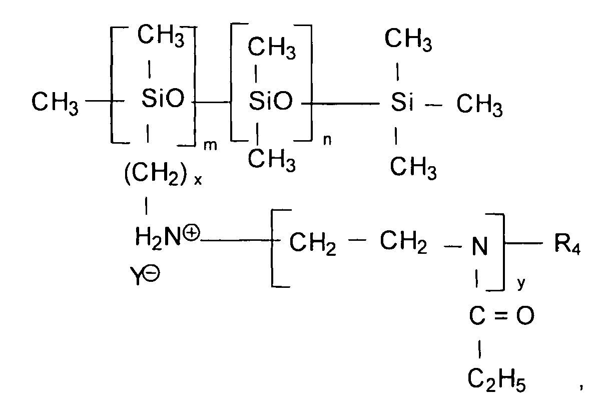 wherein m and n each are numbers from 20 to 10,000, in particular 50 to 7,000, especially 100 to 5,000, x is a number between 1 and 5, preferably 3, and y is a number from 5 to 30, R₄ is a C₁-C₁₂-alkyl or aryl group, in particular a methyl, ethyl or benzyl group, and Y- is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05% to 5%, preferably 0.1% to 2.5%, in particular 0.5% to 1.5% by weight, calculated to the total of each composition.

Cationic surfactants suitable for the third composition of the present invention are according to the general formula 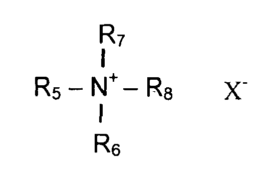 where R₅ is a saturated or unsaturated, branched or straight alkyl chain with 8-22 C atoms or

R₉ CO NH (CH₂)ₙ

where R₉ is saturated or unsaturated, branched or straight alkyl chain with 7-21 C atoms and n has value of 1 - 4,
or

R₁₀ CO O (CH₂)ₙ

where R₁₀ is saturated or unsaturated, branched or straight alkyl chain with 7-21 C atoms and n has value of 1 - 4, and
R₆ is hydrogen or unsaturated or saturated, branched or straight alkyl chain with 1 - 22 C atoms or

R₉ CO NH (CH₂)ₙ

or

R₁₀ CO O (CH₂)ₙ

where R₉, R₁₀ and n are same as above.

R₇ and R₈ are hydrogen or lower alkyl chain with 1 to 4 carbon atoms, and X is anion such as chloride, bromide, methosulfate.

Typical examples of those ingredients are cetyltrimethyl ammonium chloride, steartrimonium chloride, behentrimonium chloride, stearamidopropyl trimonuim chloride, dioleoylethyl dimethyl ammonium methosulfate, dioleoylethyl hydroxyethylmonium methosulfate.

Amido amines may as well be used as a conditioning cationic surfactant in the compositions of the present invention. Typical non-limiting example is stearamidopropylamine known with a trade name Tego Amid S18 from Degussa and behenamidopropyl dimethyl amine available under the trade name Amidet APA 22 from Kao Chemicals.

Oily substances are selected from such as silicone oils, volatile or non-volatile, natural and synthetic oils. Among silicone oils those can be added to the compositions include dimethicone, dimethiconol, polydimethylsiloxane, DC fluid ranges from Dow Corning, arylated silicones such as phenyl trimethicone or any other silicone with up to 5 aryl, preferably phenyl, group in its molecule, natural oils such as olive oil, almond oil, avocado oil, wheatgerm oil, ricinus oil and the synthetic oils, such as mineral oil, isopropyl myristate, palmitate, stearate and isostearate, oleyl oleate, isocetyl stearate, hexyl laurate, dibutyl adipate, dioctyl adipate, myristyl myristate and oleyl erucate.

The third composition according to the invention may comprise additional proteins and/or protein hydrolysates such as keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those conditioners, non-ionic compounds, oil or oil like substances, cationic polymers, silicon oil and derivatives and cationic surfactants in the third composition is in the range of 0.01 - 10% by weight, preferably 0.01 - 7.5% by weight, more preferably 0.05 - 5% and most preferably 0.1 - 5% by weight calculated to the total of third composition.

In the case that the third composition is in the form of an emulsion, it comprises as an emulsion base at least one fatty alcohol or mixture of fatty alcohols with the chain length of 14 to 22 C atoms. Examples to suitable fatty alcohols, without limiting the choice, are myristyl alcohol, cetyl alcohol, stearyl alcohol, behenyl alcohol and cetostearyl alcohol. The most preferred is cetostearyl alcohol well known with its trade name Lanette O or as Lanette N in mixture with sodium cetearyl sulfate from Cognis.

The concentration of fatty alcohol(s) is in the range from 0.5 to 20%, preferably 1 to 15% by weight, calculated to total composition prior to mixing with oxidizing and bleaching and/or highlighting composition.

The third composition according to present invention comprises surfactants selected from anionic, nonionic, amphoteric (or zwiterionic) and/or cationic surfactants or their mixtures as emulsifier or solubilizer. Cationic surfactants are as well used as hair conditioners as mentioned above.

Anionic surfactants suitable within the scope of the invention are in principal known from the cleansing compositions

These are anionic surfactants of the sulfate, sulfonate, carboxylate and alkyl phosphate type, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates.

Additional anionic surfactants useful within the scope of the invention are α-olefin sulfonates or the salts thereof, and in particular alkali salts of sulfosuccinic acid semiesters, for example, the disodium salt of monooctyl sulfosuccinate and alkali salts of long-chain monoalkyl ethoxysulfosuccinates.

Suitable surfactants of the carboxylate type are alkyl polyether carboxylic acids and the salts thereof as well as alkyl amido polyether carboxylic acids and salts thereof. Such products have been known for some time and are on the market, for example, under the trade name "AKYPO^{®}" and "AKYPO-SOFT^{®}",

Further suitable anionic surfactants are also C₈-C₂₂-acyl aminocarboxylic acids or the water-soluble salts thereof. Especially preferred is N-lauroyl glutamate, in particular as sodium salt, as well as, for example, N-lauroyl sarcosinate, N-C₁₂-C₁₈-acyl asparaginic acid, N-myristoyl sarcosinate, N-oleoyl sarcosinate, N-lauroyl methylalanine, N-lauroyl lysine and N-lauroyl aminopropyl glycine, preferably in form of the water-soluble alkali or ammonium, in particular the sodium salts thereof, preferably in admixture with the above-named anionic surfactants.

It is also possible to use mixtures of several anionic surfactants.

Further surfactants in the third composition according to the invention are nonionic surfactants which are one of the preferred emulsifying surfactant within the scope of present invention. Especially suited nonionic surfactants are, for example, long-chain fatty acid mono- and dialkanolamides, such as coco fatty acid mono- or diethanolamide and myristic fatty acid mono or diethanolamide, stearic acid mono or diethanolamide. Further nonionic surfactants suited are alkyl polyglucosides with an alkyl group of 8 to 18 carbon atoms, and with 1 to 5 glucoside units. Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}", as well as fatty alcohol ethoxylates. Further nonionic surfactants preferred in the dyeing compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16": The average degree of ethoxylation thereby ranges between about 2.5 and about 25, preferably about 10 and about 20.

As further surfactant component, the compositions according to the invention can also contain amphoteric or zwitterionic surfactants. Useful as such are in particular the various known betaines such as alkyl betaines, fatty acid amidoalkyl betaines and sulfobetaines, for example, lauryl hydroxysulfobetaine; long-chain alkyl amino acids, such as cocoaminoacetate, cocoaminopropionate and sodium cocoamphopropionate and -acetate have also proven suitable.

Cationic surfactants mentioned above as conditioner and especially those with single long alkyl chain such as cetrimonium chloride and behentrimonium chloride are also suitable emulsifiers according to present invention.

The concentration of one or more emulsifiers in the third compositions is in the range from 0.1 to 15%, preferably 0.5 to 10% by weight, calculated to total of each composition.

The third composition according to the present invention can contain organic solvent. Examples of such organic solvents are benzyloxy ethanol, benzyl alcohol, phenoxy ethanol, phenoxy isopropanol, methyl phenoxy ethanol, benzyl glycerol, N-benzyl formide, N-methyl pyrrolidone, N-ethyl pyrrolidone, cinnamyl alcohol, phenethyl alcohol, p-methyl benzyl alcohol, butyl cellosolve, methyl carbitol, ethyl carbitol, propyl carbitol, butyl carbitol, diethyleneglycol, diethyl ether and dipropyleneglycol diethyl ether. Typically the concentration of those solvents can be in the range from 0.5% to 10%, preferably 0.5 - 5% by weight calculated to the total of third composition.

The third composition can contain one or more thickening agents. The thickening agents disclosed for water free bleaching and/or highlighting compositions are also suitable for the third composition at the above given concentration ranges.

Another preferred compound in the composition of present invention especially in bleaching and/or highlighting composition and in the third composition is ceramide type of compounds according to general formula 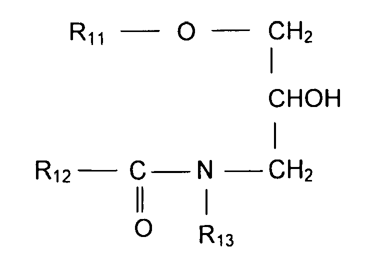 wherein R₁₁ and R₁₂ are independent from each other alkyl- or. alkenyl group with 10 to 22 carbon atoms, R₁₃ is alkyl or hydroxyl alkyl with 1 to 4 carbon atoms group and n is a number between 1 to 6, preferably 2 or 3. Preferred compound according to the above chemical structure is cetyl-PG-hydroxyethylpalmitamide. Concentration of ceramide type of compounds ranges from 0.01 to 2%, preferably 0.01 to 1% by weight calculated to total composition prior to mixing with oxidizing and bleaching and/or highlighting composition.

The third composition can also comprise one or more polyols. Suitable and preferred examples are glycerin and panthenol and their mixtures. Concentration of one or more polyols is in the range of 0.1 to 10% by weight calculated thr total of tird composition.

The third aqueous compositions can comprise at least one diamide compound. Preferred diamide compounds are according to the general structure 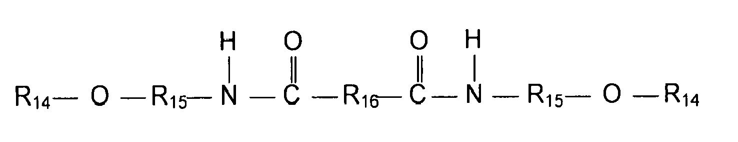 wherein R₁₄ is a linear or branched, saturated or unsaturated alkyl chain with 1 to 12 C atoms which may be substituted with hydroxy and/or alkoxy groups, preferably R₁₄ is linear or branched, saturated or unsaturated alkyl chain with 1 to 12 C atoms which may be substituted by 1 to 3 substituents selected from a hydroxy group and C1 to C6 alkoxy group, more preferably R₁₄ is a unsubstituted alkyl group with 1 to 12 C atoms, and alkyl group with 2 to 12 C atoms substituted by one or two hydroxyl groups, by one alkoxy group with 1 to 6 C atoms or by one hydroxyl and one alkoxy group with 2 to 6 C atoms, R₁₅ is linear or branched alkyl chain with 1 to 5 C atoms, preferably linear or branched alkyl chain with 2 to 5 C atoms and more preferably an alkyl chain with 2 to 3 C atoms, and R₁₆ linear or branched, saturated or unsaturated alkyl chain with 1 to 22 C atoms, preferably linear or branched, saturated or unsaturated alkyl chain with 11 to 22 C atoms.

Preferred individual diamide compounds are the ones according to the formula A to G.

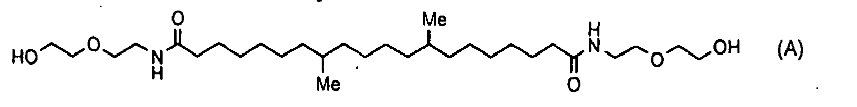

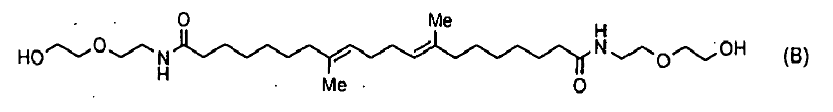

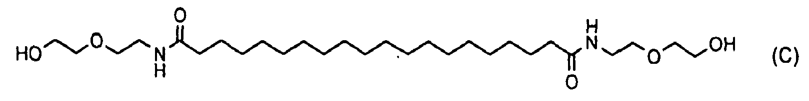

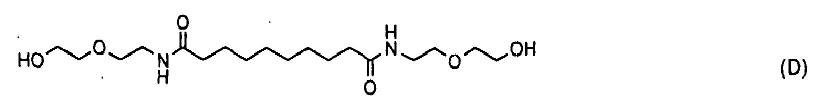

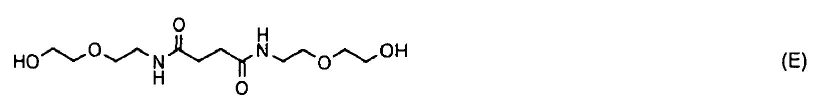

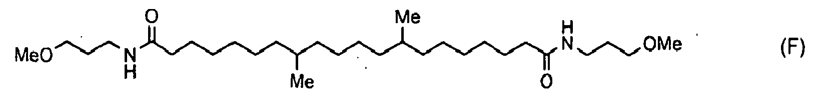

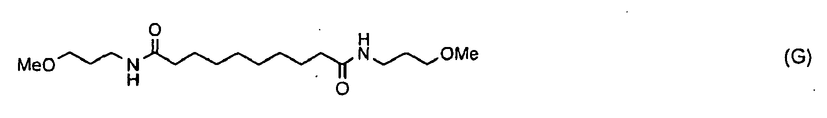

Particularly preferred diamide compound is the compound F which is bis (methoxypropylamido) isodocosane and commercially available from Kao Corporation - Japan.

Concentration of diamide compounds in the aqueous composition is in the range of 0.001 to 5%, preferably 0.002 to 3% more preferably 0.005 to 2% and most preferably 0.01 to 1% by weight calculated to total of third composition.

Another preferred compound in the composition of present invention especially in bleaching and/or highlighting composition and in the third composition is ubichinone type of compounds according to general formula 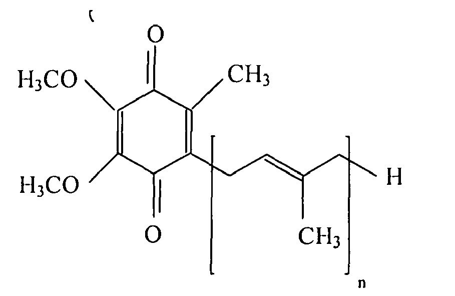 wherein n is a number from 1 to 10. Concentration of ubichinone can vary between 0.001 % and 10 % by weight, calculated to the total of each composition.

The third composition may as well comprise UV filters of oil soluble, non-ionic, ones and/or as well those of water soluble and mainly of anionic character. Non-limiting examples are Benzophenone-1 Benzophenone-2, Benzophenone-3, Benzophenone-7, Benzophenone-6, Benzophenone-8, octylmethoxy cinnamate, homosalat to those of oil soluble ones and Benzophenone-4, benzophenone-9 to those anionic water soluble ones. It should be noted that the other UV filters of oil and water soluble ones should as well be possible to combine. Concentration of UV filters is in the range of 0.05 to 5%, preferably 0.1 to 2.5% and more preferably 0.1 to 1% by weight calculated to total of third composition.

It should be noted one or more of the compounds mentioned above specifically for the third composition within the meaning of the present invention can also be comprised in the aqueous composition comprising at least one oxidizing agent so far the compound selected is compatible with the rest of the composition.
According to the preferred embodiment of the present invention, the third composition comprises at least one direct dye. Suitable direct dyes are anionic, cationic and non-ionic nitro dyes.

Suitable anionic direct dyes in aqueous composition are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium. Among those, the most preferred anionic dyestuffs are Acid Red 52, DC Violet 2, DC Red 33, DC Orange 4, DC Red 27 and DC Yellow 10.

Suitable cationic dyes in aqueous composition are in principal those available on the market for cosmetic hair colouring applications. For this purpose, special reference is made to the PCT application WO 95/15144 of Ciba-Geigy AG. Some examples to those are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 76, Basic Violet 1, Basic Violet 2, Basic Violet 3, Basic Violet 10, Basic Violet 14, Basic Yellow 57, Basic red 51, Basic Yellow 87 and Basic Orange 31. The most preferred ones are Basic red 51, Basic Yellow 87 and Basic Orange 31 sold by CIBA.

Additionally, the aqueous compositions of the present invention may comprise neutral dyes (HC dyes), so called nitro dyes for shading purposes. Some examples to those are: HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

From the above disclosed direct dyes the preferred are cationic and nitro dyes and most preferred are cationic direct dyes.

According to the invention, the third composition comprises one or more direct dye at a concentration of 0.1 to 7.5% by weight calculated to the total of third composition. The third composition can also comprise mixture of several direct dyes i.e. an anionic, a cationic and/or a nonionic ones. In such a case the dyes may be mixed at any ratio with each other.

The above mentioned direct dyes of cationic, anionic and nonionic character can also be added into the water free bleaching and/or highlighting composition at the concentration given in the above paragraph. The direct dyes of different characters can certainly be mixed as well.

pH of the aqueous third composition of the present invention varies between 2 and 12, preferably 2.5 - 10, more preferably 3 to 8, most preferably 3 to 6 and in particular 3 to 5. pH is adjusted to the required pH by using citric acid, malic acid, lactic acid, maleicacid, phosphoric acid, glycolic acid, monoethanolamine, triethanolamine, ammonia or its salts with acids such as ammonium chloride, ammonium sulphate, ammonium carbonate, ammonium bicarbonate, ammonium nitrate, or using alkaline solutions such as sodium hydroxide, potassium hydroxide and their respective salts with the known acids.

The mixing ratio of the substantially anhydrous composition comprising at least one bleaching compound to aqueous composition comprising at least one oxidizing agent to composition comprising at least one at least one compound selected from water soluble silk protein, water soluble silk protein hydrolysate and of their derivatives is in the range of 4:8:0.1 to 4:8:1, preferably 4:6:0.2 to 4:6:0.75, more preferably 4:4:0.2 to 4:4:0.75.

It should be noted that the mixing ratio of oxidizing composition and the anhydrous composition comprising at least one bleaching compound is very much dependent on the level of bleaching effect targeted, i.e. the level of highlighting and/or bleaching and darkness of hair before bleaching, and can be adjusted accordingly by hair dressers. The above mentioned ratios are general and in case somewhat different mixing ratio is needed simply because of reaching higher bleaching level than usual levels, such mixing ratio s should still be understood being within the scope of the present invention.

The pH of the ready to use product, mixture of bleaching composition and oxidizing lotion, is in the range of 8 to 12, in particular between 9 and 11.

The composition of the present invention can contain additional ingredients such as preservatives, chelating agents, fragrance and substances customarily used in cosmetic bleaching compositions of keratin fibres, especially hair.

The invention is illustrated with the following examples, but not limited to.

### Example 1

### Substantially anhydrous bleaching composition

| | % by weight |
|---|---|
| Potassium persulfate | 40 |
| Sodium persulfate | 5 |
| Sodium carbonate | 1 |
| Sodium silicate | 10 |
| Diatomaceous Earth | 40 |
| Calcium sulfate | 4 |

The above composition is prepared by combining all powder components together and by mixing until homogeneity in a suitable mixer.

### Oxidizing composition

| | % by weight |
|---|---|
| Hydrogen peroxide | 9 |
| Citric acid | q.s. to pH 3.0 |
| Water | q.s. to 100 |

### Third composition

| | % by weight |
|---|---|
| Hydrolysed silk protein* | 0.1 |
| Citric acid | q.s. to pH 4.0 |
| Preservative, fragrance | q.s. |
| Water | to 100 |

| | |
|---|---|
| *: Cosi Silk Soluble was used as commercially available. | |

The above compositions were mixed in the following formula

| | |
|---|---|
| Substantially anhydrous bleaching composition | 25 parts |
| Oxidizing composition | 35 parts |
| Third composition | 2 parts |

For comparative purposes another composition was prepared wherein the third composition was simply water (Comparative composition).

Two dark human hair tresses were bleached with the above given bleaching compositions for 40 min at 50°C. At the end of the processing time, tresses were rinsed off and shampooed once with a commercially available shampoo.

Following results were obtained:

**Table I: Results of hair bleaching**

| | **L** | **a** | **b** | Δ**E** | **Cysteic acid content (mol/100 mol)** | **Cysteic acid content increase (mol/100 mol) per level lightening** |
|---|---|---|---|---|---|---|
| Before bleaching | 18 | 1.5 | 1.0 | - | 0.7 | - |
| Inventive Composition | 56 | 11.0 | 34.0 | 52 | 5.17 | 0.086 |
| Comparative Composition | 55 | 12.0 | 33.0 | 50 | 5.42 | 0.094 |

From the above results it is clear that with the inventive composition hair tress was bleached more effectively (see ΔE and L values) and cysteic acid content of hair bleached with the inventive composition is clearly and significantly lower. This leads to the conclusion that damaging effect of inventive composition is lower compared to the comparative composition.

Similar results were observed with the following examples

### Example 2

### Bleaching / Highlighting powder composition

| | | |
|---|---|---|
| Hydroxyethylcellulose | 1.00 | % by weight |
| Cellulose gum | 3.00 | |
| Tetrasodium EDTA | 2.00 | |
| Sodium carbonate | 1.00 | |
| Ammonium persulfate | 21.00 | |
| Potassium persulfate | 46.60 | |
| Sodium metasilicate | 10.00 | |
| Diatomaceous Earth | 11.00 | |
| Polyquaternium - 10 | 0.50 | |
| Calcium chloride | 3.00 | |

### Oxidizing Lotion

| | | |
|---|---|---|
| Hydrogen peroxide | 9.00 | (% by wt.) |
| Cetyl stearyl alcohol | 1.70 | |
| Phosphoric acid | 0.50 | |
| Sodium lauryl sulfate | 0.20 | |
| Coenzyme Q10 | 0.05 | |
| Cetyl PG hydroxyethyl palmitamide | 0.10 | |
| Disodium hydrogen phosphate | 0.10 | |
| Salicylic acid | 0.10 | |
| Water | ad 100.00 | |

### Third composition

| | % by weight |
|---|---|
| Hydrolysed silk protein | 0.1 |
| Cetearyl alcohol | 3 |
| Cetrimonium chloride | 1 |
| Citric acid | to pH 3.5 |
| Preservative, Fragrance | q.s. |
| Water | to 100 |

Hydrolysed silk protein used was the same commercially available ingredient.

The compositions were mixed in the same way as in Example 1.

Dark human hair was bleached with the composition thus prepared and observed that bleached hair was easy to comb.

### Example 3

### The third composition with dyestuff

| | % by weight |
|---|---|
| | I |
| Cocamide MEA | 4.00 |
| Cetearyl alcohol | 10.00 |
| Tegin P | 1.40 |
| Propylene Glycol | 2.40 |
| Oleic acid | 3.00 |
| Hydrolysed silk protein | 0.10 |
| Ammonium chloride | 0.50 |
| Tetrasodium EDTA | 0.20 |
| Sodium lauryl sulfate | 1.50 |
| Polysilicone-9 | 0.20 |
| Pentaphenyl trimethyl trisiloxane | 5.00 |
| Cetyl PG hydroxyethyl palmitamide | 0.10 |
| Basic red 51 | 0.50 |
| Water | to 100 |

Hydrolysed silk protein used was the same commercially available ingredient.

Substantially anhydrous bleaching composition and oxidizing composition of Example 2 were used. The compositions were mixed in the same way as in Example 1.

Dark human hair was bleached in parts (streak) with the bleaching and also colouring composition of this example. Bleaching was carried out for 30 min at 40°C, and streaks were rinsed off and hair was shampooed. Intensive highlighted red streaks were obtained.

Similar results were observed when other red cationic, anionic and/or nonionic nitro dyes mentioned in the description are used instead of the cationic dye in the example.

### Example 4

### Third composition

| | |
|---|---|
| Cetrimonium chloride | 2.0 |
| Hyd roxyethylcellu lose | 0.8 |
| Cetearyl alcohol | 6.0 |
| Hydrolysed silk protein | 0.25 |
| Fragrance | q.s. |
| Preservative | q.s. |
| Citric acid | q.s. to pH 5.0 |
| Water | to 100 |

Hydrolysed silk protein used was the same commercially available ingredient.

Substantially anhydrous bleaching composition and oxidizing composition of Example 2 were used. The compositions were mixed at a weight ratio of 1:1.9:0.1 (water free composition: oxidizing lotion: the third composition). The resulting composition was tested against a bleaching composition which comprises the same amount of water instead of the third composition. It was observed that the half side bleached with a composition mixed with third composition was much more cared in terms of combability, shine and softness.

### Example 5

### Bleaching / Highlighting powder composition

| | | |
|---|---|---|
| Hydroxyethylcellulose | 1.40 | % by weight |
| Cellulose gum | 3.20 | |
| Xanthan gum | 0.30 | |
| Tetrasodium EDTA | 2.00 | |
| Sodium carbonate | 1.00 | |
| Ammonium persulfate | 21.00 | |
| Potassium persulfate | 46.60 | |
| Sodium metasilicate | 10.20 | |
| Corn starch | 1.10 | |
| Diatomaceous Earth | 9.30 | |
| Calcium sulphate | 2.80 | |
| Polyquaternium - 10 | 0.10 | |
| Silica* | 1.00 | |
| Synthetic fluorphologopite** | 1.00 | |

| | | |
|---|---|---|
| *: Aerosil 380 **: Synthetic fluorphologopite used is commercially available from Merck with a particle size distribution in the range of 5 to 45 µm. | | |

### Oxidizing Lotion

| | | |
|---|---|---|
| Hydrogen peroxide | 9.00 | (% by wt.) |
| Cetyl stearyl alcohol | 1.70 | |
| Phosphoric acid | 0.50 | |
| Sodium lauryl sulfate | 0.20 | |
| Coenzyme Q10 | 0.05 | |
| Cetyl PG hydroxyethyl palmitamide | 0.10 | |
| Disodium hydrogen phosphate | 0.10 | |
| Salicylic acid | 0.10 | |
| Water | ad 100.00 | |

### Third composition

| | |
|---|---|
| Cetrimonium chloride | 2.0 |
| Hydroxyethylcellulose | 0.8 |
| Cetearyl alcohol | 6.0 |
| Diamide compound* | 0.2 |
| Hydrolysed silk protein | 0.25 |
| Basic red 51 | 0.05 |
| HC red 3 | 0.05 |
| Acid red 52 | 0.03 |
| Basic red 76 | 0.08 |
| Fragrance | q.s. |
| Preservative | q.s. |
| Citric acid | q.s. to pH 5.0 |
| Water | to 100 |

| | |
|---|---|
| * Bis (methoxypropylamido) isodocosane and commercially available from Kao Corporation - Japan. | |

Hydrolysed silk protein used was the same commercially available ingredient.

The above composition was produced in the same way as in Example 4 and also used in the same way as described under Example 4. Intensive shiny red streaks were obtained. It was also found out that hair streaks felt less damaged upon touching which was supported by better combability, better shine and softer feel.

The same compositions used in another bleaching process wherein dyes in the third compositions were excluded. In a half side test the inventive composition was tested against comparative composition wherein only water was added instead of the third composition. It was found out that the side bleached with inventive composition was better lightened and the hair felt also softer upon touching, was easier to comb through and had better elasticity.

## Claims

1. Aqueous composition for bleaching hair comprising three parts which are mixed prior to application onto hair **characterised in that** it comprises
a- a substantially anhydrous composition comprising at least one compound with bleaching effect,
b- an aqueous composition comprising at least one oxidizing agent, and
c- a composition comprising at least one compound selected from water soluble silk protein, water soluble silk protein hydrolysate and of their derivatives.

2. Composition according to claim 1 **characterised in that** the third composition comprises water soluble silk protein hydrolysate at a concentration of 0.001 to 2.5% by weight calculated to total of third composition.

3. Composition according to claims 1 and 2 **characterised in that** substantially anhydrous composition comprises at least one compound with bleaching effect at a concentration of 5 to 85% by weight calculated to total of substantially anhydrous composition.

4. Composition according to an of the preceding claims **characterized in that** substantially anhydrous composition comprises at least one ammonium salt at a concentration of 0.1 to 10% by weight calculated to total of substantially anhydrous composition.

5. Composition according to any of the preceding claims **characterized in that** it comprises one or more of ingredients selected from lipophilic compounds, polymers and calcium salts.

6. Composition according to any of the preceding claims **characterised in that** the third composition comprises at least one conditioning agent.

7. Composition according to claims 6 **characterised in that** the third composition comprises cationic conditioning agent selected from cationic polymers and cationic surfactants or their mixtures.

8. Composition according to claims 6 to 7 **characterized in that** lipophilic conditioning agent of the third composition is selected from silicone oil, mineral oil and natural oil and their mixtures.

9. Composition according to any of the preceding claims **characterised in that** the third composition comprises one or more compounds selected from surfactant selected from anionic, non-ionic and amphoteric ones, organic solvent, ceramide compound, diamide compound, polyol, thickening agent, fatty alcohol, ubichinone, UV filter, sequestering agents, fragrance, organic and/or inorganic acids, alkaline compounds and dyestuff.

10. Composition according to any of the preceding claims **characterised in that** the third composition has a pH between 2 and 12.

11. Composition according to any of the preceding claims **characterised in that** substantially anhydrous composition, oxidizing composition and the third composition are mixed at a weight ratio between 4:8:0.1 and 4:8:1.

12. Composition according to any of the preceding claims **characterized in that** pH of the ready to use aqueous bleaching composition after mixing the three composition is between 8 and 12.

13. Use of composition according to any of the preceding claims for reducing hair damage.

14. Process for bleaching hair **characterized in that** an aqueous bleaching composition is prepared by mixing three compositions wherein
a- a substantially anhydrous composition comprising at least one compound with bleaching effect,
b- an aqueous composition comprising at least one oxidizing agent, and
c- a composition comprising at least one compound selected from water soluble silk protein, water soluble silk protein hydrolysate and of their derivatives,
is applied onto hair and after processing of 5 to 45 min at a temperature in the range of 20 to 45°C is rinsed off from hair.

15. Kit for bleaching hair which comprises 3 compositions wherein the composition are
a- a substantially anhydrous composition comprising at least one compound with bleaching effect,
b- an aqueous composition comprising at least one oxidizing agent, and
c- a composition comprising at least one compound selected from water soluble silk protein, water soluble silk protein hydrolysate and of their derivatives.
